(19) Europäisches Patentamt
European Patent Office
Office européen des brevets

(11) **EP 2 714 118 B1**

(12) **EUROPÄISCHE PATENTSCHRIFT**

(45) Veröffentlichungstag und Bekanntmachung des Hinweises auf die Patenterteilung:
**31.08.2016 Patentblatt 2016/35**

(21) Anmeldenummer: **12724538.9**

(22) Anmeldetag: **24.05.2012**

(51) Int Cl.:
***A61M 1/00*** *(2006.01)*

(86) Internationale Anmeldenummer:
**PCT/CH2012/000117**

(87) Internationale Veröffentlichungsnummer:
**WO 2012/162848 (06.12.2012 Gazette 2012/49)**

(54) **ADAPTIVER ALGORITHMUS FÜR DIE THORAXDRAINAGETHERAPIE**

ADAPTIVE ALGORITHM FOR THORACIC DRAINAGE THERAPY

ALGORITHME ADAPTATIF POUR LA THÉRAPIE DE DRAINAGE DU THORAX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.05.2011 CH 9092011**

(43) Veröffentlichungstag der Anmeldung:
**09.04.2014 Patentblatt 2014/15**

(60) Teilanmeldung:
**15161582.0 / 2 905 038**

(73) Patentinhaber: **Medela Holding AG**
**6340 Baar (CH)**

(72) Erfinder:
• **LINDER, Albert**
  **58093 Hagen (DE)**
• **WALTI, Martin**
  **CH-8038 Zürich (CH)**
• **EHLERT, Hilmar**
  **CH-6052 Hergiswil (CH)**

(74) Vertreter: **Detken, Andreas**
  **Isler & Pedrazzini AG**
  **Gotthardstrasse 53**
  **Postfach 1772**
  **8027 Zürich (CH)**

(56) Entgegenhaltungen:
EP-A2- 1 894 584        WO-A1-2009/005424
WO-A2-01/21129          US-A- 4 654 029
US-A1- 2011 071 415

**Beschreibung**

TECHNISCHES GEBIET

Die vorliegende Erfindung betrifft eine Vorrichtung für die Thoraxdrainage sowie ein entsprechendes Verfahren und Computerprogramm.

STAND DER TECHNIK

Bei Patienten mit Defekten an der Lungenoberfläche können Luftverluste im Thorax entstehen, die zur Ansammlung von Luft im Pleuraraum führen. Ein Defekt, der zum Eintritt von Luft in den Pleuraraum führt, wird im Folgenden allgemein als Luftfistel bezeichnet. Luftfisteln werden in der Regel mit einer Thoraxdrainage (Pleuraldrainage) behandelt, bei der über einen in den Pleuraraum führenden Katheter ein Vakuum an den Pleuraraum angelegt wird.

Eine wichtige Grösse für die Charakterisierung der Luftfistel ist die Luftmenge, die pro Zeiteinheit durch das Drainagesystem abgesaugt wird. Dieser Volumenstrom wird im Folgenden auch als Fistelvolumen bezeichnet. Die Grösse dieses Volumenstroms ändert sich abhängig von der funktionellen Grösse des Defektes an der Lungenoberfläche.

Das primäre Ziel bei der Behandlung von Patienten mit Luftfisteln ist in der Regel die Minimierung der Zeitdauer, während derer die Luftfistel besteht. Eine solche Minimierung geht sekundär insbesondere mit einer Reduktion der Dauer der Thoraxdrainage, des mit der Drainage einhergehenden Infektionsrisikos, der Krankenhaus-Aufenthaltsdauer und des für die Krankenbehandlung erforderlichen ärztlichen, pflegerischen und technischen Aufwandes einher.

Eine Grösse, die den Heilungsprozess einer Luftfistel positiv oder negativ beeinflussen kann, ist die Höhe des Vakuums, das an den Pleuraraum angelegt wird. Bis heute gibt es für die Regelung des Vakuums von Thoraxdrainagesystemen bei Luftfisteln jedoch keine einheitlichen Behandlungsempfehlungen. Meist beruht die Einstellung des Drucks auf Erfahrungswerten des Arztes und wird vom Arzt anlässlich der Visiten am Krankenbett ein- bis zweimal täglich individuell und manuell angepasst.

In der US 5,738,656 wird ausgeführt, dass das Vakuum eines Thoraxdrainagesystems dann optimal sei, wenn es zu einem maximalen Volumenstrom der entfernten Luft führt. Dabei sei zu beachten, dass der Volumenstrom bei zunehmendem Vakuum auch wieder abnehmen kann, weil bei einem zu hohen Vakuum z.B. periphere Lungenteile oder Koagulate das Absauglumen blockieren können. In der klinischen Praxis werden tatsächlich jedoch meist geringere Vakuumwerte gewählt. In der Regel wird das Vakuum vom Arzt manuell so eingestellt, dass die Lunge gerade ausgedehnt ist und der Volumenstrom möglichst gering ist.

In der WO 2009/005424 A1 wird vorgeschlagen, in Abhängigkeit von der Höhe des Vakuums und vom Volumenstrom von einem aktiven Betriebsmodus in einen passiven Betriebsmodus umzuschalten. Im aktiven Betriebsmodus wird ein Vakuum an den Pleuraraum angelegt. Im passiven Betriebsmodus ist der Pleuraraum über einen Sekretsammelbehälter und ein Ventil direkt mit der Atmosphäre verbunden.

In der EP 1 894 584 A2 wird vorgeschlagen, bei einem Thoraxdrainagegerät sowohl die Höhe des Vakuums als auch den Volumenstrom zu messen und anzuzeigen.

DARSTELLUNG DER ERFINDUNG

Es ist eine Aufgabe der vorliegenden Erfindung, eine Vorrichtung zur Thoraxdrainage anzugeben, die eine verbesserte, insbesondere objektivere patientenspezifische Einstellung des Vakuums erlaubt. Diese Aufgabe wird durch eine Vorrichtung nach Anspruch 1 gelöst. Weitere Ausführungsformen sind in den abhängigen Ansprüchen angegeben.

Die vorliegende Erfindung stellt also in einem ersten Aspekt eine Vorrichtung für die Thoraxdrainage bei Patienten mit einer Luftfistel zur Verfügung, wobei die Vorrichtung aufweist:

eine Saugeinrichtung zum Erzeugen eines Vakuums im Pleuraraum; sowie
eine Steuerungseinrichtung, um die Saugeinrichtung zu steuern.

Die Vorrichtung weist mindestens eine Messeinrichtung auf, die es der Steuerungseinrichtung ermöglicht, ein Mass für die Grösse der Luftfistel zu ermitteln. Die Steuerungseinrichtung ist dann dazu ausgebildet, die Höhe des von der Saugeinrichtung erzeugten Vakuums in Abhängigkeit von diesem Grössenmass zu regeln.

Während im Stand der Technik die Höhe des Absaugvakuums in der Regel manuell durch den Arzt festgelegt wird, schlägt die vorliegende Erfindung vor, ein geeignetes Mass für die funktionelle Grösse der Luftfistel zu ermitteln und anhand dieser Grösse das Absaugvakuum automatisch einzustellen. Unter dem Begriff "funktionelle Grösse" ist dabei der effektive Querschnitt zu verstehen, durch den Luft in den Pleuraraum eintritt. Indem ein Mass für die funktionelle Grösse der Luftfistel ermittelt wird, wird der Heilungsverlauf objektivierbar, und indem diese Grösse zur Steuerung der Saugeinrichtung herangezogen wird, erfolgt die Steuerung der Saugeinrichtung automatisch anhand des objektiven Heilungsverlaufs. Auf diese Weise kann der Heilungsverlauf nicht nur besser dokumentiert, sondern unter Umständen auch signifikant verkürzt werden.

Dabei ist die Steuerungseinrichtung derart ausgebildet, dass sie im Betrieb ein Verfahren mit den folgenden Schritten ausführt:

(a) Ermitteln eines ersten Wertes des Grössenmasses für die Grösse der Luftfistel;
(b) Verändern des Vakuums um einen ersten Differenzwert;
(c) Ermitteln eines zweiten Wertes des Grössenmasses nach einer ersten Wartezeit;
(d) Verändern des Vakuums um einen zweiten Differenzwert, der ein umgekehrtes Vorzeichen gegenüber dem ersten Differenzwert hat, falls der zweite Wert des Grössenmasses grösser als der erste Wert des Grössenmasses ist;
(e) Wiederholen der Schritte (a)-(d) nach einer zweiten Wartezeit.

Insbesondere kann die Steuerungseinrichtung in Schritt (b) das Vakuum um den ersten Differenzwert erhöhen und entsprechend in Schritt (d) das Vakuum gegebenenfalls um den zweiten Differenzwert senken. Die Vorrichtung überwacht in diesem Fall also selbsttätig mit einem adaptiven Algorithmus, ob die Luftfistel unter Anwendung des um den ersten Differenzwerts erhöhten Vakuums funktionell grösser oder kleiner wird. Falls das Mass für die funktionelle Grösse der Luftfistel grösser geworden ist, folgert die Vorrichtung daraus, dass der gewählte Vakuumwert zu hoch war, und reduziert diesen Wert um den zweiten Differenzwert. Andernfalls wird das Vakuum unverändert belassen. Auf diese Weise können sowohl Luftfisteln, deren funktionelle Grösse unter einem erhöhten Vakuum abnimmt ("schliessende Fisteln"), als auch Luftfisteln, deren Grösse unter einem erhöhten Vakuum zunimmt ("öffnende Fisteln"), automatisch erkannt und optimal behandelt werden. Selbstverständlich kann alternativ in Schritt (b) das Vakuum auch zunächst gesenkt werden und entsprechend in Schritt (d) erhöht werden, falls die Luftfistel unter Anwendung des in Schritt (b) gesenkten Vakuums funktionell grösser wird.

Um die automatische Regelung zu bewirken, weist die Steuerungseinrichtung vorzugsweise einen digitalen Prozessor und einen Speicher auf, in dem ein Computerprogramm gespeichert ist, welches bei Ausführung durch den Prozessor bewirkt, dass die Steuerungseinrichtung eine entsprechende automatische Regelung ausführt.

Die Messeinrichtung zur Ermittlung des Grössenmasses für die Luftfistel umfasst bevorzugt eine geeignete Druckmesseinrichtung zur Messung des angelegten Vakuums, wie sie an sich aus dem Stand der Technik bekannt ist, und/oder eine geeignete Durchflussmesseinrichtung zur Messung des Volumenstroms, wie sie ebenfalls an sich aus dem Stand der Technik bekannt ist. Eine Thoraxdrainage-Vorrichtung mit einer derartigen Druckmesseinrichtung und einer derartigen Durchflussmesseinrichtung wird z.B. unter der Bezeichnung Thopaz™ von der Medela AG, Baar, Schweiz angeboten. Das Grössenmass für die Luftfistel wird also indirekt aus dem Druck und/oder dem Volumenstrom ermittelt. Wenn eine Durchflussmesseinrichtung vorhanden ist, misst diese dabei vorzugsweise direkt den durch die Saugeinrichtung hindurch tretenden Volumenstrom. Es ist grundsätzlich aber auch denkbar, den Volumenstrom indirekt zu bestimmen, z.B. durch eine Messung der Druckdifferenz zwischen einem proximalen und einem distalen Ende des Absauglumens, durch welches die Luft aus der Pleuraraum abgesaugt wird, woraus sich bei bekanntem Strömungswiderstand des Absauglumens der Volumenstrom berechnen lässt. Ein solches Verfahren ist z.B. in US 5,738,656 näher beschrieben.

Es ist grundsätzlich denkbar, das Grössenmass für die Luftfistel alleine aus Messwerten des Volumenstroms oder alleine aus Druckmessungen zu ermitteln. Ein Verfahren, das zur Ermittlung eines Masses für die Grösse einer Luftfistel allein Druckmessungen heranzieht, wird z.B. in US 2011/0071415 vorgeschlagen. Dort wird ein Mass für die Grösse der Luftfistel ermittelt, indem die zeitliche Änderung des Druckes im Pleuraraum gemessen wird. Insbesondere wird in diesem Dokument vorgeschlagen, das Integral des Druckes über die Zeit als Mass für die Grösse der Luftfistel heranzuziehen.

Vorzugsweise werden aber sowohl das Vakuum als auch der Volumenstrom bei der Ermittlung des Grössenmasses für die Luftfistel berücksichtigt. Dazu führt die Steuerungseinrichtung vorzugsweise die folgenden Schritte aus:

Ermitteln eines Messwerts für das im Pleuraraum erzeugte Vakuum mit der Druckmesseinrichtung;
Ermitteln eines Messwerts für den Volumenstrom durch die Luftfistel mit der Durchflussmesseinrichtung;
Berechnen des Grössenmasses aus den Messwerten für den Volumenstrom und das Vakuum.

Die Steuerungseinrichtung verknüpft in diesem Fall also die Messwerte des Volumenstroms und des Vakuums mathematisch miteinander, um so auf die funktionelle Grösse der Luftfistel zu schliessen. Dies ermöglicht eine genauere Bestimmung der Grösse der Luftfistel als bei Verfahren, die allein auf den Druck oder auf den Volumenstrom abstellen. Dem liegt die Erkenntnis zu Grunde, dass ein höheres Vakuum bei konstanter Fistelgrösse auch zu einem höheren Volumenstrom führt. Indem der Volumenstrom und das Vakuum zueinander in Beziehung gesetzt werden, wird es möglich, diesen Zusammenhang zu berücksichtigen. Auf diese Weise kann ein Mass für die Fistelgrösse berechnet werden, das im Ergebnis weniger stark vom angelegten Vakuum abhängt als z.B. alleine der Volumenstrom.

Eine solche Bestimmung eines Masses für die Grösse der Luftfistel ist auch unabhängig von einer automatischen Regelung des Vakuums vorteilhaft. So kann die Vorrichtung z.B. eine Displayeinrichtung aufweisen, um das Mass für die Grösse der Luftfistel als diagnostische Grösse optisch anzuzeigen (als Zahl und/oder grafisch). Alternativ oder zusätzlich kann die Vorrichtung einen Speicher aufweisen, um das Mass für die Grösse der Luftfistel zu speichern. Die Vorrichtung kann auch ein Interface aufweisen, um das Mass für die Grösse der Luftfistel auszulesen. Jede dieser Massnahmen ermöglicht es dem behandelnden medizinischen Personal, den Behandlungsverlauf besser zu objektivieren, als dies im Stand der Technik der Fall ist.

Insbesondere ist es bevorzugt, wenn das Grössenmass auf eine solche Weise berechnet wird, dass das Ergebnis zumindest näherungsweise vom angelegten Vakuum unabhängig ist. Dazu ist die Steuerungseinrichtung vorzugsweise dazu ausgebildet, das Grössenmass für die Luftfistel zu berechnen, indem eine Variable gebildet wird, die im Wesentlichen eine Funktion des Quotients aus dem Messwert für den Volumenstrom und der Quadratwurzel des Messwerts für das Vakuum ist. Dem liegt die Erkenntnis zu Grunde, dass der Quotient des Volumenstroms über der Wurzel des Vakuumdrucks in der Regel direkt proportional zur Querschnittsgrösse der Luftfistel ist.

Insbesondere kann die Berechnung des Grössenmasses wie folgt erfolgen:

$$F = c \log_n \left( \frac{Q}{\sqrt{p}} \right),$$

wobei

F das Grössenmass für die Luftfistel ist,
Q der Messwert für den Volumenstrom ist,
p der Messwert für das Vakuum ist,
c eine Konstante, vorzugsweise c = 1, ist, und
n eine positive reelle Zahl, vorzugsweise n = 5, ist.

Auf diese Weise wird ein dimensionsloser Indikator F bereitgestellt, der eine sehr einfache Abschätzung der Fistelgrösse erlaubt.

Der Begriff "Vakuum" ist in diesem Dokument immer als negative Druckdifferenz gegenüber dem Atmosphärendruck zu verstehen. Unter dem Begriff "Erhöhen des Vakuums" ist zu verstehen, dass der Absolutwert dieser negativen Druckdifferenz vergrössert wird. Unter "Senken des Vakuums" ist zu verstehen, dass der Absolutwert der negativen Druckdifferenz verkleinert wird. Alle Angaben von Druckwerten beziehen sich im Folgenden auf die jeweiligen Absolutwerte.

Übliche Werte für das anfänglich angelegte Vakuum liegen in der Regel bei ca. 10-50 mbar (1-5 kPa), wobei dieser Bereich im Einzelfall auch verlassen werden kann. Der erste Differenzwert, um den das Vakuum im Schritt (b) des vorgeschlagenen Verfahrens jeweils erhöht oder gesenkt wird, liegt vorzugsweise zwischen 2 und 10 mbar, besonders bevorzugt zwischen 4 und 6 mbar, und kann insbesondere ca. 5 mbar betragen. Der zweite Differenzwert ist vorzugsweise grösser als der erste Differenzwert, vorzugsweise um einen Faktor zwischen 1.5 und 3 grösser als der erste Differenzwert, und kann insbesondere ungefähr doppelt so gross wie der erste Differenzwert sein. In anderen Worten wird das Vakuum im Schritt (d) vorzugsweise auf einen Wert gesenkt, der niedriger ist als der Wert, der herrschte, bevor das Vakuum im Schritt (b) erhöht worden war, falls in Schritt (b) eine Erhöhung erfolgte. Falls in Schritt (b) alternativ eine Senkung erfolgte, wird das Vakuum in Schritt (d) vorzugsweise auf einen Wert erhöht, der höher ist als der Wert, der vor dem Schritt (b) herrschte. In absoluten Zahlen liegt der zweite Differenzwert vorzugsweise zwischen 5 und 20 mbar, besonders bevorzugt zwischen 8 und 12 mbar, und beträgt insbesondere ca. 10 mbar.

Die erste Wartezeit liegt vorzugsweise zwischen 20 min und 3 h, besonders bevorzugt zwischen 40 min und 1.5 h, und kann insbesondere ca. 1 h betragen. Die zweite Wartezeit ist bevorzugt grösser als die erste Wartezeit, vorzugsweise um einen Faktor zwischen 2 und 5 grösser als die erste Wartezeit, und kann insbesondere um einen Faktor von ca. 3 grösser als die erste Wartezeit sein. Die zweite Wartezeit liegt vorzugsweise zwischen 1 h und 6 h, besonders bevorzugt zwischen 2 h und 4 h, und kann insbesondere ca. 3 h betragen.

Die genannten Druckdifferenzen und Wartezeiten sind so gewählt, dass mehrmals täglich eine Anpassung erfolgt, und dass innerhalb einer überschaubaren Beobachtungsdauer (z.B. innerhalb einer Schicht des Pflegepersonals) eine Veränderung der Grösse der Luftfistel aufgrund der veränderten Druckverhältnisse wahrscheinlich ist.

Die erste und/oder zweite Wartezeit müssen nicht notwendigerweise fix vorgegeben sein, sondern können gegebenenfalls auch dynamisch angepasst werden. Insbesondere kann z.B. die erste Wartezeit verkürzt werden, falls das Mass für die Fistelgrösse nach dem Erhöhen des Vakuumwertes über einen vorgegebenen Alarmwert hinaus ansteigt. In diesem Fall ist eine umgehende Reduktion des Vakuums angezeigt. Die Steuerungseinheit kann entsprechend dazu ausgebildet sein, die Einhaltung eines solchen Alarmwerts zu überwachen und gegebenenfalls bei Überschreiten des Alarmwerts zusätzlich ein Alarmsignal abzugeben.

Die Vorrichtung weist bevorzugt weitere Merkmale auf, wie sie bei Thoraxdrainagesystemen allgemein üblich sind, insbesondere einen Sekretsammelbehälter, durch den die von der Saugeinrichtung abgesaugte Luft hindurch geleitet wird, um flüssige und feste Körpersekrete abzuscheiden. Zudem kann in der üblichen Weise ein Wasserschloss vorgesehen sein, um ein Zurückfliessen von Luft in den Pleuraraum zu verhindern. Die Vorrichtung wird in der Regel zumindest einen ersten Schlauchanschluss zum Anschliessen eines Absaugschlauches oder Absaugkatheters aufweisen. Ausserdem kann die Vorrichtung einen zweiten Schlauchanschluss zum Anschliessen eines Hilfsschlauchs oder Hilfskatheter aufweisen. Der Absaugschlauch und der Hilfsschlauch sind dann zumindest an ihren patientennahen (proximalen) Enden miteinander verbunden, und die durch die Schläuche begrenzten Lumina kommunizieren miteinander. Insbesondere kann die Druckmesseinrichtung mit dem zweiten Schlauchanschluss verbunden sein, so dass die Druckmessung über den Hilfsschlauch in einem patientennahen Bereich, also nahe dem Pleuraraum, erfolgt, ohne durch die Strömung der abgesaugten Luft und Körpersekrete verfälscht zu werden.

Ausserdem wird ein Verfahren für die Thoraxdrainage bei einem Patienten mit einer Luftfistel offenbart, mit den folgenden Schritten:

Erzeugen eines Vakuums im Pleuraraum mittels einer Saugeinrichtung;
Ermitteln eines Grössenmasses für die Luftfistel; und
automatisches, insbesondere computergesteuertes, Regeln des von der Saugeinrichtung erzeugten Vakuums in Abhängigkeit von diesem Grössenmass.

Zudem wird ein Verfahren für die Thoraxdrainage offenbart, bei dem ein Vakuum an den Pleuraraum eines Patienten mit einer Luftfistel angelegt wird, wobei das Verfahren die oben genannten Schritte (a)-(e) aufweist. Dieses Verfahren kann manuell ausgeführt werden, z.B. durch einen Arzt oder durch Pflegepersonal. Bevorzugt wird das Verfahren aber zumindest teilweise automatisch durchgeführt, d.h. mindestens die Schritte (a)-(d) werden selbsttätig durch eine Steuerungseinrichtung, insbesondere computergesteuert, durchgeführt.

Ausserdem wird ein Verfahren für die Thoraxdrainage bei Patienten mit einer Luftfistel offenbart, bei dem ein Grössenmass für die Luftfistel wie folgt ermittelt wird:

Ermitteln eines Messwerts für das im Pleuraraum erzeugte Vakuum;,
Ermitteln eines Messwerts für den Volumenstrom durch die Luftfistel; und
Berechnen des Grössenmasses für die Luftfistel aus den Messwerten für den Volumenstrom und das Vakuum.

Das so ermittelte Grössenmass kann gespeichert oder ausgegeben werden und/oder zur Regelung des Vakuums herangezogen werden.

Die vorliegende Erfindung bezieht sich ausserdem auf ein Computerprogramm mit Code zur Steuerung einer Vorrichtung für die Thoraxdrainage bei Patienten mit einer Luftfistel, wobei die Vorrichtung eine Saugeinrichtung zum Erzeugen eines Vakuums sowie eine digitale Steuerungseinrichtung zur Steuerung der Saugeinrichtung umfasst, wobei der Code, wenn er in der digitalen Steuerungseinrichtung ausgeführt wird, die Steuerungseinrichtung eines der oben genannten Verfahren durchrühren lässt.

Das Computerprogramm kann insbesondere in Form eines Computerprogrammprodukts auf einem geeigneten Datenträger vorliegen, z.B. auf einer CD-ROM, auf einem Flashspeicher usw., oder über ein Netzwerk zum Download bereitgestellt werden. Es kann in beliebiger Form vorliegen, z.B. als Source Code, Object Code oder Maschinencode.

Für das Verfahren und das Computerprogramm gelten ansonsten sinngemäss die selben Überlegungen wie für die Vorrichtung, insbesondere was die Ermittlung und Berechnung des Grössenmasses für die Luftfistel und die Überlegungen zu den Werten der ersten und zweiten Druckdifferenz und zu den Wartezeiten betrifft. Dabei kann das Verfahren auch mit anderen Mitteln als mit der oben beschriebenen Vorrichtung ausgeführt werden.

KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

Fig. 1     eine Prinzipskizze für eine Thoraxdrainage;
Fig. 2     eine beispielhafte Absaugvorrichtung, wobei das Gehäuse teilweise durchbrochen dargestellt ist, um einen Einblick in das Innere des Gehäuses zu erlauben;
Fig. 3     eine Prinzipskizze zur Illustration der Funktionsweise der Absaugvorrichtung der Fig. 2; und
Fig. 4     einen Ablaufplan für ein erfindungsgemässes Verfahren.

EP 2 714 118 B1

BESCHREIBUNG BEVORZUGTER AUSFÜHRUNGSFORMEN

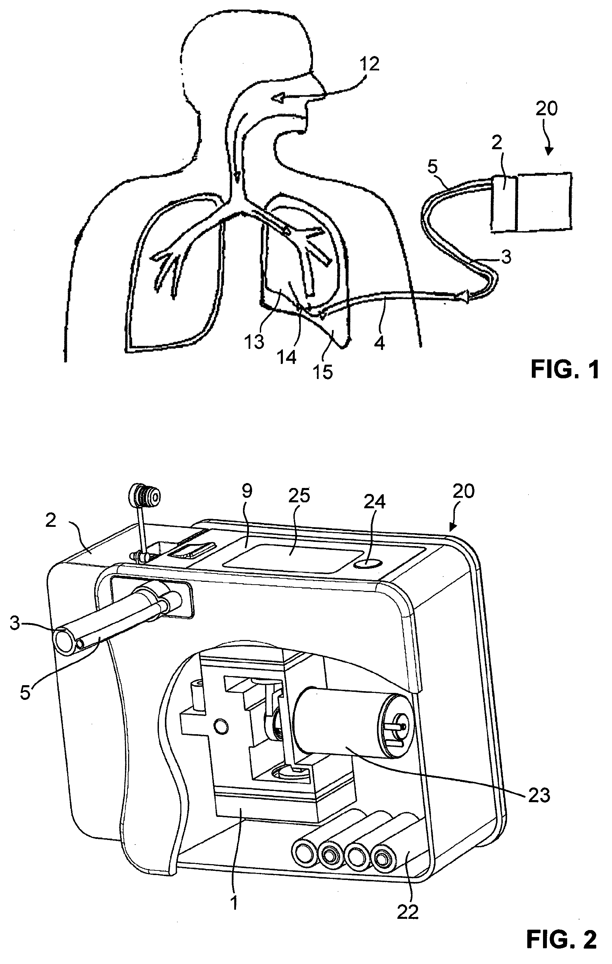

Die Fig. 1 illustriert schematisch das Prinzip einer Thoraxdrainage. Ein Patient weist eine Luftfistel 14 zwischen einem Lungenflügel 13 und dem Pleuraraum 15 auf, die z.B. spontan, durch eine Verletzung oder iatrogen entstanden sein kann. Durch die Luftfistel gelangt Luft von der Lunge in den Pleuraraum 15. Um die Luft abzusaugen, erstreckt sich ein Katheter 4 in den Pleuraraum hinein. Dieser ist über ein Schlauchsystem mit Absaugschlauch 3 und Hilfsschlauch 5 mit einer Absaugvorrichtung 20 mit austauschbarem Sammelbehälter 2 verbunden. Der Sammelbehälter dient dazu, Körpersekrete, die zusammen mit der abgesaugten Luft zur Absaugvorrichtung 20 gelangen, abzuscheiden.

Ein Beispiel für eine geeignete und an sich bekannte Absaugvorrichtung ist in der Fig. 2 illustriert. Die Absaugvorrichtung 20 weist eine Saugpumpe 1 auf, die durch einen Elektromotor 23 angetrieben wird. Der Elektromotor 23 wird durch eine Steuerungseinrichtung 9 angesteuert. Die Steuerungseinrichtung 9 verfügt über Bedien- und Anzeigeelemente in Form eines Hauptschalters 24 und eines Touchscreen-Displays 25. Die Steuerungseinrichtung und damit auch der Elektromotor werden von einem Energiespeicher in Form von aufladbaren Batterien 22 mit Strom versorgt, so dass die Absaugvorrichtung autonom und tragbar ist. Die Absaugvorrichtung der Fig. 2 ist im Detail in der WO 2007/128156 beschrieben, deren Inhalt durch Verweis in vollem Umfang in diese Beschreibung aufgenommen wird. Eine Absaugvorrichtung von grundsätzlich ähnlichem Aufbau ist unter dem Namen Medela Thopaz™ kommerziell erhältlich.

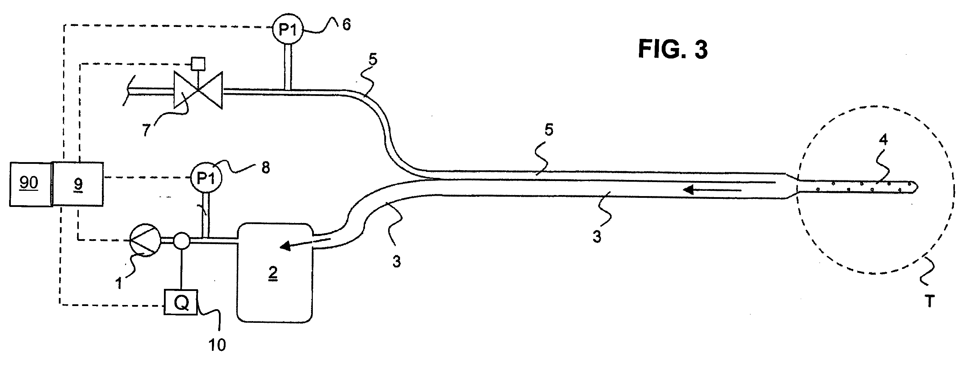

Die Funktionsweise einer solchen Absaugvorrichtung ist schematisch in der Fig. 3 illustriert. Der Katheter 4 befindet sich mit seinem proximalen Ende im Pleuraraum T. Der Katheter ist mit dem proximalen Ende des Absaugschlauchs 3 und des Hilfsschlauchs 5 verbunden. An seinem distalen Ende mündet der Absaugschlauch 3 in den Sammelbehälter 2. Dieser ist mit der Saugpumpe 1 verbunden, um ein Vakuum im Sammelbehälter 2 zu erzeugen. Zwischen dem Sammelbehälter 2 und der Saugpumpe 1 sind eine erste Druckmesseinrichtung 8 sowie eine Durchflussmesseinrichtung 10 angeschlossen, die den Druck im Sammelbehälter 2 sowie den Gasvolumenstrom durch den Sammelbehälter 2 hindurch messen. Das distale Ende des Hilfsschlauchs ist mit einem steuerbaren Ventil 7 sowie einer zweiten Druckmesseinrichtung 6 verbunden. Bei geschlossenem Ventil 7 fliesst kein Gas durch den Hilfsschlauch 5, und die Druckmesseinrichtung 6 misst folglich im Wesentlichen den Druck am proximalen Ende des Hilfsschlauchs 5. Dieser Druck entspricht weitgehend dem Druck im Pleuraraum T. Die Steuerungseinrichtung 9 empfängt Signale von den Druckmesseinrichtungen 6, 8 und von der Durchflussmesseinrichtung 10 und steuert die Saugpumpe 1 sowie das Ventil 7. Für weitere Merkmale der Absaugvorrichtung der Fig. 3 sei auf die WO 2005/061025 verwiesen, deren Inhalt hiermit durch Verweis in vollem Umfang in die vorliegende Beschreibung aufgenommen wird.

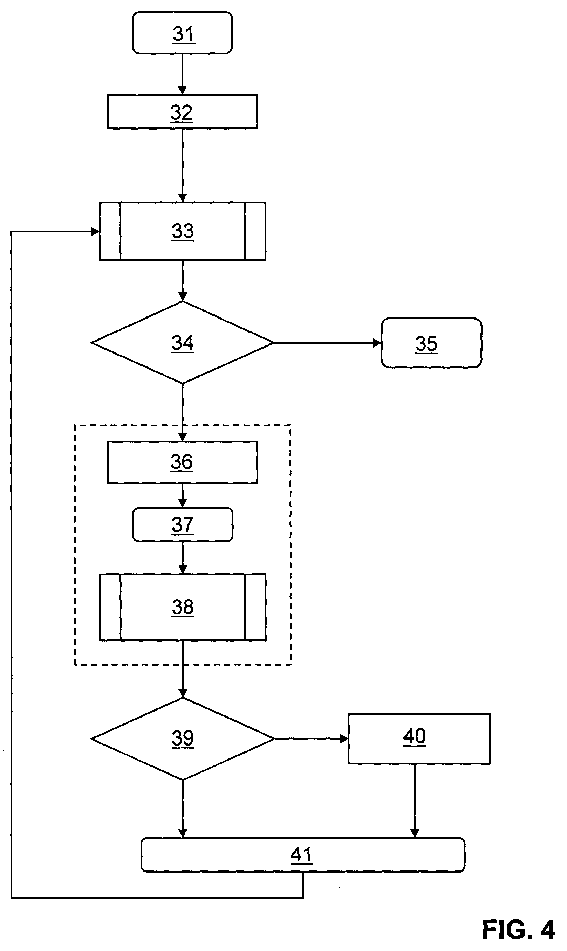

In einem bevorzugten Ausführungsbeispiel handelt es sich bei der Steuerungseinrichtung 9 um eine digitale Steuerungseinrichtung, d.h. diese Einrichtung umfasst einen digitalen Prozessor und einen damit zusammenwirkenden Speicher 90, in dem ein Computerprogramm zur Ausführung durch den Prozessor geladen ist. Wenn das Programm abläuft, führt die Steuerungseinrichtung 9 den in der Fig. 4 illustrierten adaptiven Algorithmus aus, welcher im Folgenden Schritt für Schritt erläutert wird.

*Schritt 31:* Start. In diesem Schritt wird die Absaugvorrichtung 20 in Betrieb genommen und ein Anfangsvakuum eingestellt.

*Schritt 32:* Dokumentation Anfangsvakuum. Das Anfangsvakuum wird mit einem Zeitstempel versehen und im Speicher 90 der Steuerungseinrichtung 9 zu Dokumentationszwecken abgelegt.

*Schritt 33:* Ermitteln eines ersten Werts der Fistelgrösse. Die Steuerungseinrichtung 9 ermittelt einen ersten Wert eines Masses für die Grösse der Luftfistel. Dazu misst die Steuerungseinrichtung mittels der zweiten Druckmesseinrichtung 6 den im Pleuraraum anliegenden Wert des Vakuums und misst mittels der Durchflussmesseinrichtung 10 den vom Pleuraraum her kommenden Volumenstrom durch den Absaugschlauch 3. Daraus ermittelt die Steuerungseinrichtung ein Grössenmass für die Fistel gemäss der folgenden Gleichung:

$$F = c \log_n \left( \frac{Q}{\sqrt{p}} \right),$$

wobei

$F$ das Grössenmass für die Luftfistel ist,
$Q$ der Messwert für den Volumenstrom (ausgedrückt in Millilitern pro Minute) ist,
$p$ der Messwert für das Vakuum (ausgedrückt in mbar) ist, und wobei
$c = 1$ und $n = 5$.

Das Ergebnis ist in der Regel eine Zahl zwischen 0 und 5. Diese Zahl wird mit einem Zeitstempel versehen und im Speicher der Steuerungseinrichtung 90 abgelegt, ebenso die Werte $Q$ und $p$.

*Schritt 34:* Test Fistelgrösse. Das Grössenmass wird mit einem vorbestimmten Referenzwert (der insbesondere gleich 1 sein kann) verglichen.

*Schritt 35:* Ende. Falls das Grössenmass kleiner oder gleich dem Referenzwert ist, wird ein entsprechendes Endsignal abgegeben, das den Patienten oder das medizinische Personal darauf hinweist, dass die Luftfistel praktisch geschlossen ist und die Thoraxdrainage voraussichtlich beendet werden kann.

*Schritt 36:* Vakuum um einen ersten Differenzwert erhöhen. Falls das Grössenmass den Referenzwert übersteigt, erhöht die Steuerungseinrichtung 9 die Pumpleistung derart, dass das Vakuum um 5 mbar ansteigt. Der neue Vakuumwert wird mit Zeitstempel im Speicher 90 abgelegt.

*Schritt 37:* erste Wartezeit. In dieser Zeit (z.B. eine Stunde) wird der neue Vakuumwert beibehalten. Die Luftfistel erhält Gelegenheit, auf den neuen Vakuumwert zu reagieren.

*Schritt 38:* Ermitteln des zweiten Werts der Fistelgrösse. Dieser Schritt läuft genau wie Schritt 33 ab.

*Schritt 39:* Vergleich Fistelgrössen vorher/nachher: Der erste und der zweite Wert des Grössenmasses werden aus dem Speicher 90 ausgelesen und miteinander verglichen. Wenn der zweite Wert grösser ist als der erste Wert, wird nach Schritt 40 verzweigt, andernfalls nach Schritt 41.

*Schritt 40:* Senken des Vakuums um einen zweiten Differenzwert. Falls der zweite Wert grösser ist als der erste Wert des Fistelmasses, verringert die Steuerungseinrichtung die Leistung der Saugpumpe, bis das Vakuum um 10 mbar gesunken ist. Andernfalls wird das bisherige Vakuum beibehalten. Der neue Vakuumwert wird mit Zeitstempel im Speicher 90 abgelegt.

*Schritt 41:* zweite Wartezeit: In dieser Zeit (3 Stunden) wird das Vakuum wiederum beibehalten.

Die Schritte 33, 34, 36-39 und 41 sowie gegebenenfalls Schritt 40 werden nun auf gleiche Weise wiederholt, bis das Verfahren bei Schritt 35 endet. Die gespeicherten Werte für das Vakuum, den Volumenstrom und das Grössenmass können jederzeit über eine Schnittstelle ausgelesen oder auf dem Display dargestellt werden.

Durch die im Vergleich zur bisher üblichen klinischen Praxis häufigen Anpassungen des Vakuums kann der Heilungsverlauf besser überwacht und idealerweise beschleunigt werden.

*Beispiel 1: "schliessende Fistel"*

Bei einem Patienten 1 mit einer Luftfistel wurde das Thoraxdrainagesystem Thopaz™ eingesetzt. Anfänglich wurde ein Vakuum von 10 mbar eingestellt. Dies resultierte in einem Volumenstrom von 100 ml/min. Hieraus wurde mit der obigen Gleichung (siehe Schritt 33) eine Fistelgrösse $F$ = 2.15 berechnet. Das Vakuum wurde nun um 5 mbar auf 15 mbar erhöht. Nach einer Stunde wurde erneut der Volumenstrom gemessen. Dieser betrug nun 120 ml/min. Daraus wurde eine Fistelgrösse $F$ = 2.13 berechnet. Die Fistel hatte sich also funktionell verkleinert. Das Vakuum von 15 mbar wurde daher für weitere 3 Stunden beibehalten.

*Beispiel 2: "öffnende Fistel"*

Bei einem Patienten 2 wurde ebenfalls ein Anfangsvakuum von 10 mbar eingestellt. Auch bei diesem Patienten resultierte ein Volumenstrom von 100 ml/min, woraus sich eine Fistelgrösse $F$ = 2.15 ergab. Das Vakuum wurde wiederum um 5 mbar auf 15 mbar erhöht, und nach einer Stunde wurde der Volumenstrom gemessen. Dieser betrug nun 240 ml/min, entsprechend einer Fistelgrösse $F$ = 2.56. Das Vakuum wurde daher um 10 mbar auf nur noch 5 mbar gesenkt, und dieser Wert wurde für 3 Stunden beibehalten.

Es ist offensichtlich, dass eine Vielzahl von Abwandlungen der vorstehend beispielhaft beschriebenen Vorrichtungen und Verfahren möglich sind. So kann es sich bei der Saugeinrichtung um eine beliebige andere Art von Saugeinrichtung als die elektrische Saugpumpe der Fig. 2 handeln, z.B. um einen Anschluss für ein zentrales Krankenhaus-Vakuumsystem, so lange die Vakuumhöhe durch einen geeigneten Drucksteller einstellbar ist. Dementsprechend kann das Thoraxdrainagesystem auch vollkommen anders aufgebaut sein als in den Figuren 2 und 3. Aus dem Stand der Technik sind eine Vielzahl von derartigen Systemen bekannt.

Auch der adaptive Algorithmus kann auf vielfältige Weise abgewandelt oder ergänzt werden. So können insbesondere die Differenzwerte des Vakuums und die Wartezeiten auch anders gewählt werden, z.B. dynamisch an Messwerte angepasst werden. Dabei können ausser der Fistelgrösse auch noch andere Parameter, z.B. die Menge an flüssigem Sekret, automatisch gemessen und zur automatischen Regelung des Vakuums herangezogen werden.

Grundsätzlich ist es möglich, das Verfahren der Schritte 31-41 vollkommen manuell durchzuführen, auch wenn eine automatisierte Durchführung bevorzugt ist.

BEZUGSZEICHENLISTE

| 1 | Saugpumpe | 12 | Luft |
|---|---|---|---|
| 2 | Sekretbehälter | 13 | Lungenflügel |
| 3 | Absaugschlauch | 14 | Luftfistel |
| 4 | Katheter | 15 | Pleuraraum |
| 5 | Hilfsschlauch | 20 | Absaugvorrichtung |
| 6 | zweite Druckmesseinrichtung | 22 | Batterien |
| 7 | Ventil | 23 | Motor |
| 8 | erste Druckmesseinrichtung | 24 | Hauptschalter |
| 9 | Steuerungseinrichtung | 25 | Touchscreen-Display |
| 10 | Durchflussmesseinrichtung | 31-41 | Verfahrensschritte |

**Patentansprüche**

1.  Vorrichtung für die Thoraxdrainage bei Patienten mit einer Luftfistel (14), aufweisend:

    eine Saugeinrichtung (1) zum Erzeugen eines Vakuums;
    eine Steuerungseinrichtung (9), um die Saugeinrichtung (1) zu steuern; sowie
    mindestens eine Messeinrichtung (6, 10), die es der Steuerungseinrichtung (9) ermöglicht, ein Grössenmass für die Luftfistel (14) zu ermitteln,

    dadurch gekennzeichnet, dass die Steuerungseinrichtung (9) dazu ausgebildet ist, ein Verfahren mit den folgenden Schritten auszuführen, um die Höhe des von der Saugeinrichtung (1) erzeugten Vakuums in Abhängigkeit von diesem Grössenmass zu regeln:

    (a) Ermitteln eines ersten Wertes des Grössenmasses;
    (b) Verändern des Vakuums um einen ersten Differenzwert;
    (c) Ermitteln eines zweiten Wertes des Grössenmasses nach einer ersten Wartezeit;
    (d) Verändern des Vakuums um einen zweiten Differenzwert, der ein umgekehrtes Vorzeichen gegenüber dem ersten Differenzwert hat, falls der zweite Wert des Grössenmasses grösser als der erste Wert des Grössenmasses ist;
    (e) Wiederholen der Schritte (a)-(d) nach einer zweiten Wartezeit.

2.  Vorrichtung nach Anspruch 1, wobei der erste Differenzwert einen Absolutwert zwischen 2 und 10 mbar aufweist.

3.  Vorrichtung nach Anspruch 1 oder 2, wobei der zweite Differenzwert einen Absolutwert aufweist, der grösser als der Absolutwert des ersten Differenzwerts ist.

4.  Vorrichtung nach einem der Ansprüche 1-3, wobei die erste Wartezeit zwischen 20 min und 3 h beträgt.

5.  Vorrichtung nach einem der Ansprüche 1-4, wobei die zweite Wartezeit grösser als die erste Wartezeit ist.

6.  Vorrichtung nach einem der vorhergehenden Ansprüche, wobei die Messeinrichtung eine Druckmesseinrichtung (6) zur Messung des erzeugten Vakuums und/oder eine Durchflussmesseinrichtung (10) zur Messung eines Volumenstroms umfasst.

7.  Vorrichtung nach Anspruch 6, wobei die Messeinrichtung sowohl eine Druckmesseinrichtung (6) zur Messung des erzeugten Vakuums als auch eine Durchflussmesseinrichtung (10) zur Messung eines Volumenstroms umfasst,

**dadurch gekennzeichnet, dass** die Steuerungseinrichtung (9) dazu ausgebildet ist, das Grössenmass für die Luftfistel (14) zu ermitteln, indem sie die folgenden Schritte ausführt:

Ermitteln eines Messwerts für das im Pleuraraum (15) erzeugte Vakuum mit der Druckmesseinrichtung (6);
Ermitteln eines Messwerts für den Volumenstrom durch die Luftfistel (14) mit der Durchflussmesseinrichtung (10);
Berechnen des Grössenmasses aus den Messwerten für den Volumenstrom und das Vakuum.

8. Vorrichtung nach Anspruch 7, wobei die Steuerungseinrichtung (9) dazu ausgebildet ist, das Grössenmass für die Luftfistel (14) zu berechnen, indem eine Variable gebildet wird, die eine Funktion des Quotients aus dem Messwert für den Volumenstrom und der Quadratwurzel des Messwerts für das Vakuum ist.

9. Vorrichtung nach Anspruch 8, wobei die Steuerungseinrichtung (9) dazu ausgebildet ist, das Grössenmass für die Luftfistel wie folgt zu berechnen:

$$F = c \log_n \left( \frac{Q}{\sqrt{p}} \right),$$

wobei

$F$ das Grössenmass für die Luftfistel ist,
$Q$ der Messwert für den Volumenstrom ist,
$p$ der Messwert für das Vakuum ist,
$c$ eine Konstante, vorzugsweise $c = 1$, ist, und
$n$ eine positive reelle Zahl, *vorzugsweise* $n = 5$, ist.

10. Computerprogramm zur Steuerung einer Vorrichtung für die Thoraxdrainage bei Patienten mit einer Luftfistel (14), wobei die Vorrichtung eine Saugeinrichtung (1) zum Erzeugen eines Vakuums sowie eine digitale Steuerungseinrichtung (9) zur Steuerung der Saugeinrichtung umfasst, wobei das Computerprogramm, wenn es in der digitalen Steuerungseinrichtung (9) ausgeführt wird, die Steuerungseinrichtung veranlasst, ein Grössenmass für die Luftfistel (14) zu ermitteln und **dadurch gekennzeichnet** ist, ein Verfahren mit den folgenden Schritten auszuführen, um die Höhe des von der Saugeinrichtung (1) erzeugten Vakuums in Abhängigkeit von diesem Grössenmass zu regeln:

(a) Ermitteln eines ersten Wertes eines Grössenmasses für die Luftfistel (14);
(b) Verändern des Vakuums um einen ersten Differenzwert;
(c) Ermitteln eines zweiten Wertes des Grössenmasses nach einer ersten Wartezeit;
(d) Verändern des Vakuums um einen zweiten Differenzwert, der ein umgekehrtes Vorzeichen gegenüber dem ersten Differenzwert hat, falls der zweite Wert des Grössenmasses grösser als der erste Wert des Grössenmasses ist;
(e) Wiederholen der Schritte (a)-(d) nach einer zweiten Wartezeit.

11. Computerprogramm nach Anspruch 10,
**dadurch gekennzeichnet, dass** das Computerprogramm, wenn es in der digitalen Steuerungseinrichtung (9) ausgeführt wird, die Steuerungseinrichtung veranlasst, das Grössenmass für die Luftfistel (14) zu ermitteln, indem es die folgenden Schritte ausführt:

Ermitteln eines Messwerts für das erzeugte Vakuum;
Ermitteln eines Messwerts für den Volumenstrom durch die Luftfistel (14); und
Berechnen des Grössenmasses für die Luftfistel (14) aus den Messwerten für den Volumenstrom und das Vakuum.

12. Computerprogramm nach Anspruch 11, wobei das Computerprogramm, wenn es in der digitalen Steuerungseinrichtung (9) ausgeführt wird, die Steuerungseinrichtung veranlasst, das Grössenmass für die Luftfistel zu berechnen, indem es eine Variable bildet, die eine Funktion des Quotients aus dem Messwert für den Volumenstrom und der Quadratwurzel des Messwerts für das Vakuum ist.

**13.** Computerprogramm nach Anspruch 12, wobei das Computerprogramm, wenn es in der digitalen Steuerungseinrichtung (9) ausgeführt wird, die Steuerungseinrichtung veranlasst, das Grössenmass für die Luftfistel wie folgt zu berechnen:

$$F = c \log_n(\frac{Q}{\sqrt{p}}) \, ,$$

wobei

*F* das Grössenmass für die Luftfistel ist,
*Q* der Messwert für den Volumenstrom ist,
*p* der Messwert für das Vakuum ist,
*c* eine Konstante ist, und
*n* eine positive reelle Zahl ist.

## Claims

**1.** An appliance for thoracic drainage in patients with an air fistula (14), said appliance comprising:

a suction device (1) for generating a vacuum;
a control device (9) for controlling the suction device (1), and
at least one measuring device (6, 10), which allows the control device (9) to determine a size parameter for the air fistula (14),

**characterized in that** the control device (9) is configured to carry out a method having the following steps, in order to regulate the level of the vacuum generated by the suction device (1) as a function of said size parameter:

(a) determining a first value of the size parameter;
(b) changing the vacuum by a first differential value;
(c) determining a second value of the size parameter after a first waiting period;
(d) changing the vacuum by a second differential value, which has an opposite sign compared to the first differential value, if the second value of the size parameter is greater than the first value of the size parameter;
(e) repeating steps (a)-(d) after a second waiting period.

**2.** The appliance according to Claim 1, wherein the first differential value has an absolute value of between 2 and 10 mbar.

**3.** The appliance according to Claim 1 or 2, wherein the second differential value has an absolute value that is greater than the absolute value of the first differential value.

**4.** The appliance according to one of Claims 1-3, wherein the first waiting period is between 20 minutes and 3 hours.

**5.** The appliance according to one of Claims 1-4, wherein the second waiting period is longer than the first waiting period.

**6.** The appliance according to one of the preceding claims, wherein the measuring device comprises a pressure gauge (6) for measuring the generated vacuum and/or a flow meter (10) for measuring a volumetric flow.

**7.** The appliance according to Claim 6, wherein the measuring device comprised both a pressure gauge (6) for measuring the generated vacuum and a flow meter (10) for measuring a volumetric flow, **characterized in that** the control device (9) is configured to determine the size parameter for the air fistula (14) by carrying out the following steps:

using the pressure gauge (6) to determine a measured value for the vacuum generated in the pleural space (15);
using the flow meter (10) to determine a measured value for the volumetric flow through the air fistula (14);
calculating the size parameter from the measured values for the volumetric flow and the vacuum.

**8.** The appliance according to Claim 7, wherein the control device (9) is configured to calculate the size parameter for the air fistula (14) by forming a variable which is a function of the quotient from the measured value for the volumetric flow and the square root of the measured value for the vacuum.

9. The appliance according to Claim 8, wherein the control device (9) is configured to calculate the size parameter for the air fistula as follows:

$$F = c \log_n \left( \frac{Q}{\sqrt{p}} \right),$$

where

F is the size parameter for the air fistula,
Q is the measured value for the volumetric flow,
p is the measured value for the vacuum,
c is a constant, preferably c = 1, and
n is a positive real number, preferably n = 5.

10. A computer program for controlling an appliance for thoracic drainage in patients with an air fistula (14), said appliance comprising a suction device (1) for generating a vacuum and a digital control device (9) for controlling the suction device, wherein the computer program, when executed in the digital control device (9), causes the control device to determine a size parameter for the air fistula (14) and is characterized to carry out a method comprising the following steps, in order to regulate the level of the vacuum generated by the suction device (1) as a function of said size parameter:

(a) determining a first value of a size parameter for the air fistula (14);
(b) changing the vacuum by a first differential value;
(c) determining a second value of the size parameter after a first waiting period;
(d) changing the vacuum by a second differential value, which has an opposite sign compared to the first differential value, if the second value of the size parameter is greater than the first value of the size parameter; and
(e) repeating steps (a)-(d) after a second waiting period.

11. The computer program according to Claim 10, **characterized in that** the computer program, when executed in the digital control device (9), causes the control device to determine the size parameter for the air fistula (14) by carrying out the following steps:

determining a measured value for the vacuum generated;
determining a measured value for the volumetric flow through the air fistula (14); and
calculating the size parameter for the air fistula (14) from the measured values for the volumetric flow and the vacuum.

12. The computer program according to Claim 11, wherein the computer program, when executed in the digital control device (9), causes the control device to calculate the size parameter for the air fistula by forming a variable which is a function of the quotient from the measured value for the volumetric flow and the square root of the measured value for the vacuum.

13. The computer program according to Claim 12, wherein the computer program, when executed in the digital control device (9), causes the control device to calculate the size parameter for the air fistula as follows:

$$F = c \log_n \left( \frac{Q}{\sqrt{p}} \right),$$

where

F is the size parameter for the air fistula,
Q is the measured value for the volumetric flow,
p is the measured value for the vacuum,

*c* is a constant, and
*n* is a positive real number.

**Revendications**

1. Dispositif pour le drainage du thorax chez un patient présentant une fistule à air (14), comprenant :

   un appareil d'aspiration (1) pour la création d'un vide ;
   un appareil de contrôle (9) pour contrôler l'appareil d'aspiration (1), ainsi que au moins un appareil de mesure (6,10), qui permet à l'appareil d'e contrôle (9) de déterminer un ordre de grandeur pour la fistule à air (14), **caractérisé en ce que** l'appareil de contrôle (9) est agencé pour effectuer un procédé ayant les étapes suivantes pour régler le niveau du vide généré par l'appareil d'aspiration (1) en fonction de cet ordre de grandeur :

   (a) déterminer une première valeur de l'ordre de grandeur ;
   (b) modifier le vide d'une première valeur différentielle ;
   (c) déterminer une deuxième valeur de l'ordre de grandeur après un premier temps d'attente ;
   (d) modifier le vide d'une deuxième valeur différentielle ayant un signe inversé par rapport à la première valeur différentielle, si la deuxième valeur de l'ordre de grandeur est supérieure à la première valeur de l'ordre de grandeur ;
   (e) répéter les étapes (a)-(d) après un deuxième temps d'attente.

2. Dispositif selon la revendication 1, dans lequel la première valeur différentielle a une valeur absolue entre 2 et 10 mbar.

3. Dispositif selon la revendication 1 ou 2, dans lequel la deuxième valeur différentielle présente une valeur absolue qui est supérieure à la valeur absolue de la première valeur différentielle.

4. Dispositif selon une des revendications 1 à 3, dans lequel le premier temps d'attente est entre 20 minutes et 3 heures.

5. Dispositif selon une des revendications 1 à 4, dans lequel le deuxième temps d'attente est supérieur au premier temps d'attente.

6. Dispositif selon une de revendications précédentes, dans lequel l'appareil de mesure comprend un appareil de mesure de pression (6) pour la mesure du vide créé et/ou un appareil de mesure de débit (10) pour la mesure d'un flux volumétrique.

7. Dispositif selon la revendication 6, dans lequel l'appareil de mesure comprend un appareil de mesure de pression (6) pour la mesure du vide créé ainsi qu'un appareil de mesure de débit (10) pour la mesure d'un flux volumétrique, **caractérisé en ce que** l'appareil de contrôle (9) est agencé pour déterminer un ordre de grandeur pour la fistule à air (14), en effectuant les étapes suivantes :

   (a) déterminer une valeur de mesure pour le vide créé dans l'espace pleural (15) au moyen de l'appareil de mesure de pression (6) ;
   (b) déterminer une valeur de mesure pour le flux volumétrique à travers la fistule à air (14) au moyen de l'appareil de mesure de débit (10) ;
   (c) calculer l'ordre de grandeur à partir des valeurs de mesure pour le flux volumétrique et du vide.

8. Dispositif selon la revendication 7, dans lequel l'appareil de contrôle (9) est agencé pour calculer l'ordre de grandeur pour la fistule à air (14), en formant une variable en fonction du quotient de la valeur de mesure pour le flux volumétrique et la racine carrée de la valeur de mesure pour le vide.

9. Dispositif selon la revendication 8, dans lequel l'appareil de contrôle (9) est agencé pour calculer l'ordre de grandeur de la fistule à air comme suit :

$$F = c \log_n\left(\frac{Q}{\sqrt{p}}\right),$$

dans laquelle

F est l'ordre de grandeur pour la fistule à air,
Q est la valeur de mesure pour le flux volumétrique,
p est la valeur de la mesure pour le vide,
c est une constante, préférablement c est égal à 1 et
n est un nombre positive réel, préférablement égal à 5.

10. Un programme informatique pour le contrôle d'un dispositif pour le drainage de thorax chez un patient présentant une fistule à air (14), dans lequel le dispositif comprend un appareil d'aspiration (1) pour la création d'un vide, ainsi qu'un appareil de contrôle numérique (9) pour le contrôle de l'appareil d'aspiration, où le programme informatique, lorsqu'il est effectué dans l'appareil de contrôle numérique (9) amène l'appareil de contrôle à déterminer un ordre de grandeur pour la fistule à air (14) et est **caractérisé en ce qu'**il effectue un procédé ayant les étapes suivantes, pour déterminer le niveau de vide généré par l'appareil d'inspiration (1) en fonction de cet ordre de grandeur :

(a) déterminer une première valeur de l'ordre de grandeur pour la fistule à air (14);
(b) modifier le vide d'une première valeur différentielle ;
(c) déterminer une deuxième valeur de l'ordre de grandeur après un premier temps d'attente ;
(d) modifier le vide d'une deuxième valeur différentielle ayant un signe inversé par rapport à la première valeur différentielle, si la deuxième valeur de l'ordre de grandeur est supérieure à la première valeur de l'ordre de grandeur ;
(e) répéter les étapes (a)-(d) après un deuxième temps d'attente.

11. Programme informatique selon la revendication 10, **caractérisé en ce que** le programme informatique, lorsqu'il est effectué dans l'appareil de contrôle numérique (9), amène l'appareil de contrôle à déterminer l'ordre de grandeur pour la fistule à air (14), en effectuant les étapes suivantes ;
déterminer une valeur de mesure pour le vide créé ;
déterminer une valeur de mesure pour le flux volumétrique à travers la fistule d'air (14) ; et
calculer l'ordre de grandeur pour la fistule à air (14), à partir des valeurs de mesure pour le flux volumétrique et le vide.

12. Programme informatique selon la revendication 11, où le programme informatique, lorsqu'il est effectué dans l'appareil de contrôle numérique (9), amène l'appareil de contrôle à calculer l'ordre de grandeur pour la fistule à air (14) en formant une variable en fonction du quotient de la valeur de mesure pour le flux volumétrique et la racine carrée de la valeur de mesure pour le vide.

13. Programme informatique selon la revendication 12, où le programme informatique, lorsque il est effectué dans l'appareil de contrôle numérique (9), amène l'appareil de contrôle à calculer l'ordre de grandeur pour la fistule à air comme suit :

$$F = c \log_n \left( \frac{Q}{\sqrt{p}} \right),$$

dans laquelle

F est l'ordre de grandeur pour la fistule à air,
Q est la valeur de mesure pour le flux volumétrique,
p est la valeur de la mesure pour le vide,
c est une constante, et
n est un nombre positive réel.

**FIG. 1**

**FIG. 2**

# FIG. 3

FIG. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5738656 A
- WO 2009005424 A1
- EP 1894584 A2

- US 20110071415 A
- WO 2007128156 A
- WO 2005061025 A